(19) Europäisches Patentamt
European Patent Office
Office européen des brevets

(11) Veröffentlichungsnummer : **0 141 295**
**B1**

(12) # EUROPÄISCHE PATENTSCHRIFT

(45) Veröffentlichungstag der Patentschrift : **30.12.86**

(21) Anmeldenummer : **84111998.5**

(22) Anmeldetag : **06.10.84**

(51) Int. Cl.$^4$ : **C 07 C119/042**, C 07 C119/045, C 07 C118/00, C 07 C125/06

(54) **Ungesättigte Esterisocyanate, ein Verfahren zu ihrer Herstellung und ihre Verwendung bei der Herstellung von olefinisch ungesättigten Oligourethanen.**

(30) Priorität : **20.10.83 DE 3338077**

(43) Veröffentlichungstag der Anmeldung : **15.05.85 Patentblatt 85/20**

(45) Bekanntmachung des Hinweises auf die Patenterteilung : **30.12.86 Patentblatt 86/52**

(84) Benannte Vertragsstaaten : **AT BE DE FR GB IT NL SE**

(56) Entgegenhaltungen :
**EP-A- 0 000 144**
**DE-B- 1 267 214**
**DE-B- 1 924 535**

(73) Patentinhaber : **BAYER AG**
**Konzernverwaltung RP Patentabteilung**
**D-5090 Leverkusen 1 Bayerwerk (DE)**

(72) Erfinder : **Klein, Gerhard, Dr.**
**von-Flotow-Strasse 7**
**D-4019 Monheim (DE)**
Erfinder : **Arlt, Dieter, Prof. Dr.**
**Rybniker Strasse 2**
**D-5000 Köln 80 (DE)**

**EP 0 141 295 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue, olefinisch ungesättigte Esterisocyanate, ein Verfahren zu ihrer Herstellung durch Phosgenierung der ihnen zugrunde liegenden Dihydrooxazine, sowie ihre Verwendung als Ausgangsmaterial zur Herstellung von olefinisch ungesättigten Oligourethanen.

Ungesättigte Esterisocyanate und ihre Herstellung durch Phosgenierung der ihnen zugrunde liegenden Oxazoline bzw. Dihydrooxazine sind bereits bekannt. So wird beispielsweise in der DE-AS 1 924 535 bzw. der EP-PS 144 die Herstellung von 2-Isocyanatoethylmethacrylat (IEM) durch Phosgenierung des entsprechenden Oxazolins im Detail beschrieben. In der DE-AS 1 924 535 ( = GB-PS 1 252 099) wird darüber hinaus auch beiläufig 2-Vinyl-5,6-dihydro-1,3-oxazin bzw. 2-Isopropenyl-5,6-dihydro-1,3-oxazin als geeignetes Ausgangsmaterial erwähnt, jedoch findet sich keine konkrete Lehre, wie aus diesen Ausgangsmaterialien das entsprechende Isocyanatopropylacrylat bzw. -methacrylat hergestellt werden kann. Tatsächlich ist das 2-Isocyanatoethylmethacrylat das einzige ungesättigte Esterisocyanat, das bislang technische Bedeutung erlangte.

Obwohl Isocyanatoethylmethacrylat ein vielseitig verwendbares Zwischenprodukt, insbesondere zur Herstellung von polymerisierbaren oder mit anderen olefinisch ungesättigten Monomeren copolymerisierbaren Kunststoff-Vorläufern darstellt — beispielsweise kann IEM mit Verbindungen mit mindestens zwei gegenüber Isocyanatgruppen reaktionsfähigen Gruppen, wie beispielsweise einfachen mehrwertigen Alkoholen wie Butan-1,4-diol oder Trimethylolpropan zu mindestens zwei olefinische Doppelbindungen aufweisenden Oligourethanen umgesetzt werden, die ihrerseits wertvolle Monomere oder Comonomere bei der Herstellung von Polymerisaten darstellen — weist das Zwischenprodukt auch einige Nachteile auf. Indiesem Zusammenhang sind insbesondere der hohe Dampfdruck des Zwischenprodukts, die Tatsache, daß es sich bei vielen der genannten Folgeprodukte (Umsetzungsprodukte von IEM mit einfachen mehrwertigen Alkoholen) um Festsubstanzen handelt und die oft unzureichende Verträglichkeit der genannten Folgeprodukte mit den bei der Herstellung von Polymerisationskunststoffen mitverwendeten Comonomeren zu nennen.

Die der Erfindung zugrunde liegende Aufgabe bestand darin, neue ungesättigte Esterisocyanate zur Verfügung zu stellen, die diese Nachteile nicht mehr aufweisen bzw. in denen diese Nachteile in weit weniger ausgeprägter Form vorliegen.

Diese Aufgabe konnte durch die Bereitstellung der neuen, nachstehend näher beschriebenen, ungesättigten Esterisocyanate bzw. des Verfahrens zu ihrer Herstellung gelöst werden.

Gegenstand der Erfindung sind, gegebenenfalls als Isomerengemische vorliegende, ungesättigte Esterisocyanate, dadurch gekennzeichnet, daß sie im wesentlichen

zu 0-95 Gew.-%, bezogen auf Gesamtgemisch, aus Verbindungen der Formel

$$OCN{-}CH_2{-}R_1{-}O{-}CO{-}\overset{R_2}{\overset{|}{C}}{=}CH{-}R_3 \qquad (I)$$

zu 0 bis 70 Gew.-%, bezogen auf Gesamtgemisch, aus Verbindungen der Formel

$$OCN{-}CH_2{-}R_1'{-}O{-}CO{-}\overset{R_2}{\overset{|}{C}}{=}CH{-}R_3 \qquad (II)$$

und zu 0-70 Gew.-%, bezogen auf Gesamtgemisch, aus Verbindungen der Formel

$$OCN{-}CH_2{-}R_1''{-}O{-}CO{-}\overset{R_2}{\overset{|}{C}}{=}CH{-}R_3 \qquad (III)$$

bestehen, wobei sich die genannten Prozentsätze jeweils zu 100 ergänzen und wobei

$R_1$, $R_1'$ und $R_1''$ entweder für den gleichen Rest stehen und jeweils einen gegebenenfalls Alkyl-substituierten 1,2-Cycloalkylen-Rest mit insgesamt 5 bis 10 Kohlenstoffatomen bedeuten, oder wobei $R_1$ für einen 2,3-Butylen-Rest, $R_1'$ für einen geradkettigen 1,2-Butylen-Rest und $R_1''$ für einen 1,2-Isobutylen-Rest stehen,

$R_2$ und $R_3$ für gleiche oder verschiedene Reste stehen und jeweils Wasserstoff oder eine Methylgruppe bedeuten.

Gegenstand der Erfindung ist auch ein Verfahren zur Herstellung dieser Esterisocyanate durch Umsetzung von gegebenenfalls als Säureaddukte vorliegenden Dihydrooxazinen mit Phosgen bei —20 bis + 20 °C in einem mit Wasser nicht mischbaren Lösungsmittel in Gegenwart einer wäßrigen Lösung einer Base, dadurch gekennzeichnet, daß man als Dihydrooxazine solche verwendet, die sich im wesentlichen

zu 0-95 Gew.-% aus Verbindungen der Formel

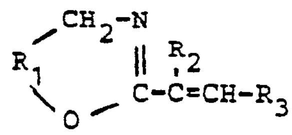

(IV)

zu 0-70 Gew.-% aus Verbindungen der Formel

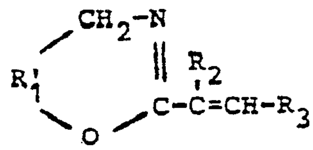

(V)

und zu 0-70 Gew.-% aus Verbindungen der Formel

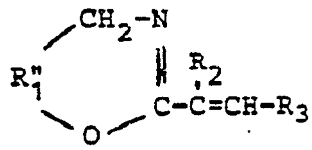

(VI)

zusammensetzen, wobei sich die Prozentsätze jeweils zu 100 ergänzen, und wobei $R_1$, $R_1'$, $R_1''$, $R_2$ und $R_3$ die obengenannte Bedeutung haben.

Gegenstand der Erfindung ist auch die Verwendung der erfindungsgemäßen Esterisocyanate als Ausgangsmaterialien bei der Herstellung von olefinisch ungesättigten Oligourethanen.

Im Rahmen der vorliegenden Erfindung sind unter « 1,2-Cycloalkylen-Rest » 2-wertige (gegebenenfalls Alkylsubstituierte) cycloaliphatische Kohlenwasserstoffreste zu verstehen, deren cycloaliphatische Ringe in 1,2-Stellung mit der Methylengruppe bzw. dem Sauerstoffatom der Esterisocyanate bzw. der Oxazine verknüpft sind. Dementsprechend sind unter «1,2-Butylen-Rest », « 2,3-Butylen-Rest » bzw. « 1,2-Isobutylen-Rest » aliphatische, 2-wertige Kohlenwasserstoffreste mit 4 Kohlenstoffatomen zu verstehen, die in 1,2- bzw. 2,3-Stellung mit der Methylengruppe bzw. dem Sauerstoff verknüpft sind, wobei, im Falle von asymmetrischen Resten, jeweils das höher substituierte Kohlenstoffatom mit dem Sauerstoffatom verknüpft ist.

Bei den für das erfindungsgemäße Verfahren als Ausgangsmaterialien geeigneten Dihydrooxazinen der oben genannten allgemeinen Formel kann es sich entweder um definierte Verbindungen ($R_1 = R_1' = R_1''$ = gegebenenfalls Alkyl-substituierter 1,2-Cycloalkylen-Rest mit insgesamt 5 bis 10 Kohlenstoffatomen) oder um, den obigen Ausführungen entsprechende, Isomerengemische ($R_1$, $R_1'$ und $R_1''$ stehen für verschiedene Butylen- bzw. Isobutylen-Reste) handeln. Die Zusammensetzung der Esterisocyanate entspricht bezüglich der Isomerenverteilung der Zusammensetzung der zu ihrer Herstellung eingesetzten Dihydrooxazine. Vorzugsweise werden folgende Dihydrooxazine als Ausgangsmaterialien beim erfindungsgemäßen Verfahren eingesetzt :

a) Verbindungen der Formel

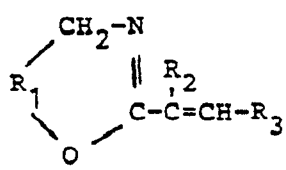

(IV)

in welcher

$R_1$ für einen 1,2-Cyclohexylen-, einen 3- (und/oder 4-)Isopropyl-1,2-cyclohexylen- oder einen 1-Methyl-4-isopropyl-1,2-cyclohexylen-Rest steht, und

$R_2$ und $R_3$ die oben genannte Bedeutung haben, wobei $R_3$ besonders bevorzugt für Wasserstoff steht.

Typische Beispiele für derartige Ausgangsmaterialien sind beispielsweise die Verbindungen der Formeln

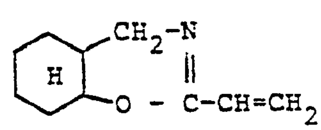

(VII)

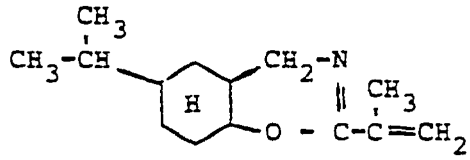

(VIII)

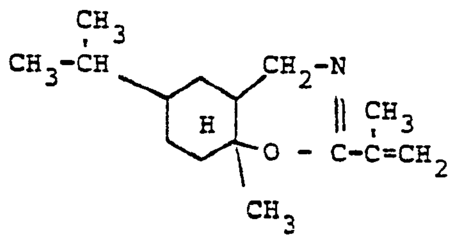

(IX)

b) Isomerengemische, die sich
zu 85-95 Gew.-% aus 2-Vinyl- bzw. 2-Isopropenyl-5,6-dimethyl-5,6-dihydrooxazin und
zu 5-15 Gew.-% aus 2-Vinyl- bzw. 2-Isopropenyl-6-ethyl-5,6-dihydrooxazin
zusammensetzen.

In diesen zuletzt genannten Isomerengemischen stellen die 2-Vinyl- bzw. 2-Propenyl-5,6-dimethyl-5,6-dihydrooxazine ihrerseits vorzugsweise cis/trans-Isomerengemische im Gewichtsverhältnis cis : trans = (33 ± 10) : (66 ± 10) dar.

Neben diesen bevorzugten Ausgangsverbindungen können beim erfindungsgemäßen Verfahren beliebige andere, der obigen breiten Definition entsprechende Dihydrooxazine bzw. Dihydrooxazin-Isomerengemische eingesetzt werden. Beispielsweise kommen auch sehr gut auch solche Dihydrooxazin-Isomerengemische in Betracht, die bis zu 70 Gew.-%, vorzugsweise bis zu 50 Gew.-% aus 2-Vinyl- bzw. 2-Isopropenyl-6,6-dimethyl-5,6-dihydrooxazin und im übrigen aus 2-Vinyl- bzw. 2-Isopropenyl-5,6-dimethyl-5,6-dihydrooxazin und/oder 2-Vinyl- bzw. 2-Isopropenyl-6-ethyl-5,6-dihydrooxazin bestehen, wobei auch hier vorzugsweise die 5,6-Dimethyl-substituierten Dihydrooxazine ihrerseits cis/trans-Isomerengemische im oben genannten Verhältnis darstellen. Weiterhin sind beim erfindungsgemäßen Verfahren solche Dihydrooxazine bzw. Dihydrooxazin-Isomerengemische der beispielhaft genannten Art verwendbar, die in 2-Stellung am Dihydrooxazin-Ring anstelle eines Vinyl- bzw. Isopropenyl-Substituenten einen n-Propenyl-Substituenten der Formel

$$—CH{=}CH—CH_3 \qquad (X)$$

aufweisen.

Die Herstellung der als Ausgangsmaterialien einzusetzenden Dihydrooxazine bzw. Dihydrooxazin-Isomerengemische erfolgt in Analogie zu den bekannten Verfahren des Standes der Technik. So können die Ausgangsverbindungen beispielsweise aus N-Hydroxy-methylamiden der allgemeinen Formel

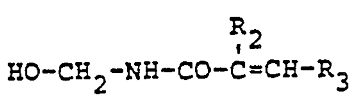

(XI)

und einen, dem Rest $R_1$ entsprechenden Olefin bzw. einem den Resten $R_1$-$R_1''$ entsprechenden Olefingemisch nach dem in Liebigs Annalen *697*, Seiten 171-180 (1966) beschriebenen Verfahren hergestellt werden.

Vorteilhafter gewinnt man sie aus Formaldehyd, einem Nitril der allgemeinen Formel

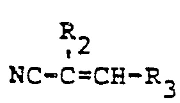

(XII)

und einem, dem Rest $R_1$ entsprechenden Olefin bzw. den Resten $R_1$-$R_1''$ entsprechenden Olefingemisch analog dem in Synthesis (1971), Seiten 92-95 beschriebenen Verfahren.

Bei diesem Verfahren wird Formaldehydin in einem Lösungsmittel in Gegenwart äquimolarer Mengen einer starken Säure in einem Temperaturbereich zwischen 30 und 100 °C, vorzugsweise bei 50-60 °C mit dem Nitril der allgemeinen Formel XII umgesetzt.

Das dabei gebildete Aminomethyliumion der Formel

$$\overset{(+)}{CH_2=NH}-CO-\overset{R_2}{\overset{|}{C}}=CH-R_3 \qquad (XIII)$$

reagiert mit dem Olefin bzw. Olefingemisch in einer polaren Cycloaddition zu dem Säureaddukt des Dihydroxazins, aus welchem das als erfindungsgemäßes Ausgangsmaterial geeignete Dihydrooxazin durch an sich bekannte Behandlung mit einer Base (vgl. nachstehende Ausführungsbeispiele) gewonnen werden kann.

Formaldehyd kann dabei entweder durch Depolymerisation aus Paraformaldehyd oder aus 1.3,5-Trioxan gewonnen werden. Als Lösungsmittel können Carbonsäuren, Carbonsäureanhydride, Ether wie z. B. Tetrahydrofuran, Dioxan, Glyme, Diglyme, Amide wie z. B. N-Methylpyrrolidon, Harnstoff, wie z. B. 1,3-Dimethylpyrrolidon-2 oder Sulfolan verwendet werden. Bevorzugt sind Carbonsäuren, insbesondere Essigsäure.

Als starke Säuren kommen Schwefelsäure, Phosphorsäure, Chlorwasserstoff, Fluorwasserstoff, Borfluorwasserstoffsäure und Sulfonsäuren in Betracht. Am vorteilhaftesten wird Schwefelsäure benutzt. In jedem Falle ist auf Wasserausschluß zu achten.

Das Nitril wird in äquimolaren Mengen in einem Temperaturbereich zwischen 30 und 100 °C, vorzugsweise bei 50-60 °C, zu einer Lösung von Formaldehyd und der starken Säure in dem Lösungsmittel gegeben. Als Olefin bzw. Olefingemisch kommen dem Rest $R_1$ bzw. den Resten $R_1$-$R_1''$ entsprechende Verbindungen in Betracht. Dies bedeutet, daß beispielsweise die Verwendung von Cyclohexen zu Dihydrooxazinen der obengenannten allgemeinen Formel IV führt, für welche $R_1$ für einen 1,2-Cyclohexylen-Rest steht. Die oben unter b) genannten, als erfindungsgemäße Ausgangsmaterialien bevorzugt geeigneten Dihydrooxazin-Isomerengemische werden beispielsweise dann erhalten, wenn als Olefingemisch ein Buten-Gemisch der ungefähren Zusammensetzung 20 Gew.-% cis-Buten-2, 37 Gew.-% trans-Buten-2, 12 Gew.-% 1-Buten, 7 Gew.-% iso-Butan und 23 Gew.-% n-Butan verwendet wird. Derartige und ähnliche technische Buten-Gemische, die neben reaktionsfähigen Butenen inerte Butane enthalten, fallen beispielsweise bei der destillativen Auftrennung der Spaltprodukte von Benzincrackern als $C_4$-Fraktion an. Andere großtechnische $C_4$-Fraktionen von Benzincrackern mit einem hohen Gehalt an isobuten gestatten die Herstellung von Dihydrooxazin-Gemischen mit einem hohen Gehalt an 6,6-Dimethyl-substituierten Isomeren. Da das in den genannten Fraktionen enthaltene 1-Buten im Vergleich zu cis- und trans-Buten-2 reaktionsträger ist, ist der Gehalt an 6-Ethyl-2-vinyl-5,6-dihydrooxazin in den Dihydrooxazin-Gemischen im allgemeinen geringer als der Gehalt der eingesetzten $C_4$-Fraktion an 1-Buten.

Die Reaktion zwischen dem Säureaddukt des Dihydrooxazins und dem Olefin bzw. Olefingemisch kann in einem offenen Gefäß unter Durchleiten oder Zutropfen des Olefins, bei gasförmigen Olefinen auch unter Druck durchgeführt werden.

Die Bildung des Dihydrooxazins aus dem Amidomethyliumion XIII und dem Olefin erfolgt in einer stereospezifischen cis-Addition (siehe Chem. Ber. *103*, 3242 (1970). Aus einem cis-/trans-Olefin-Gemisch bildet sich deshalb ein entsprechendes cis-/trans-Gemisch des 5,6-Dimethydihydrooxazins.

Wie bereits erwähnt, kann das freie Dihydrooxazin aus dem erhaltenen Säureaddukt in an sich bekannter Weise mittels einer base wie z. B. Natriumhydroxid oder Kaliumhydroxid freigesetzt werden. Es ist jedoch auch möglich, die Dihydrooxazine beim erfindungsgemäßen Verfahren in Form ihrer Säureaddukte einzusetzen, d. h. die bei der Umsetzung der Amidomethyliumionen mit dem Olefin anfallenden Säureaddukte des Dihydrooxazins stellen nach Entfernung des Hilfslösungsmittels unmittelbar beim erfindungsgemäßen Verfahren einsetzbare Ausgangsmaterialien dar.

Das erfindungsgemäße Verfahren, d. h. die Phosgenierung der Dihydrooxazine bzw. Dihydrooxazin-Gemische erfolgt vorzugsweise nach der bekannten Zweiphasen-Phosgenierung, wie sie beispielsweise in DE-AS 1 924 535 für die Phosgenierung von Oxazolinen oder von Dihydrooxazinen beschrieben ist. Dabei werden pro Mol Dihydrooxazin. bzw. pro Mol Säureaddukt des Dihydrooxazins bzw. Dihydrooxazin-Gemischs im allgemeinen 1 bis 2 Mol Phosgen und pro Mol Phosgen mindestens 2 Mol einer wäßrigen Base eingesetzt. Falls. Säureaddukte der Dihydrooxazine eingesetzt werden, benötigt man zusätzlich noch eine der Säure äquivalente Menge an Base.

Als Basen können wäßrige Lösung von Alkalihydroxiden und -carbonaten verwendet werden. Bevorzugt ist wäßrige Natronlauge. Das Dihydrooxazin und das Phosgen werden im allgemeinen als Lösungen in einem unpolaren, mit Wasser nicht mischbaren Lösungsmittel eingesetzt. Dafür kommen Kohlenwasserstoffe, Halogenkohlenwasserstoffe wie z. B. Methylenchlorid, Chloroform, 1,2-Dichlorpropan, Chlorbenzol und Dichlorbenzol, Ester wie z. B. Ethylacetat oder Ether wie Diethyl- oder Dibutylether in Frage. Am vorteilhaftesten ist die Verwendung von Halogenkohlenwasserstoffen, insbesondere von Methylenchlorid.

Die Lösungen des Dihydrooxazins, des Phosgens und der Base werden gleichzeitig und gleichmäßig dem Reaktionsgefäß zugeführt. Dabei ist für eine intensive Durchmischung zu sorgen. Die Temperatur wird bei —20 bis +20 °C, vorzugsweise zwischen 0 und 5 °C gehalten. Da die Reaktion sehr schnell abläuft, ist eine kontinuierliche Reaktionsführung zweckmäßig.

Da bei der Phosgenierungsreaktion die Stereochemie der Kohlenstoffatome 4-6 des Dihydrooxazinrings nicht geändert wird, weisen die aus den oben unter b) genannten, bevorzugten Dihydrooxazin-Gemischen hergestellten Esterisocyanate im allgemeinen folgende Zusammensetzung auf :

60 ± 10 Gew.-% threo-1-Isocyanato-2-methyl-but-3-yl-(meth)acrylat
30 ± 10 Gew.-% erythro-1-Isocyanato-2-methyl-but-3-yl-(meth)acrylat
10 ± 5 Gew.-% 1-Isocyanatopent-3-yl-(meth)acrylat.

Die erfindungsgemäßen, ungesättigten Esterisocyanate stellen u. a. wertvolle Zwischenprodukte zur Herstellung von mehrere olefinische Doppelbindungen aufweisenden Oligourethanen dar, die ihrerseits als Monomere, bzw. Comonomere, insbesondere bei der Herstellung von vernetzten Polymerisationskunststoffen verwendet werden können. Zur Herstellung derartiger, mehrfach olefinisch ungesärttigter Oligourethane werden die erfindungsgemäßen Esterisocyanate mit beliebigen Verbindungen mit gegenüber Isocyanatgruppen reaktionsfähigen Gruppen im Sinne einer Isocyanat-Additionsreaktion zur Reaktion gebracht. Die Umsetzung kann durch die üblichen Urethanisierungskatalysatoren, d. h. durch tertiäre Amine, wie z. B. Triethylendiamin, oder durch Dibutylzinndilaurat bzw. Zinndioctat beschleunigt werden.

Die Umsetzung kann in Gegenwart eines inerten Lösungsmittels, vorzugsweise jedoch ohne Lösungsmittel, in einem Temperaturbereich zwischen 20 und 120 °C, vorzugsweise zwischen 50 und 80 °C, vorgenommen werden.

Bei der Umsetzung werden die Esterisocyanate im allgemeinen in, bezogen auf die gegenüber Isocyanatgruppen reaktionsfähigen Gruppen, mindestens äquivalenten Mengen eingesetzt, wobei ein gegebenenfalls zum Einsatz gelangter Isocyanat-Überschuß nach erfolgter Umsetzung destillativ aus dem Reaktionsgemisch entfernt werden kann. Auch die Verwendung eines Isocyanat-Unterschußes ist im Prinzip möglich, um auf diese Weise olefinische Doppelbindungen und gegenüber Isocyanatgruppen reaktionsfähige Gruppen aufweisende Verbindungen herzustellen, die ihrerseits die Herstellung von Polymerisationskunststoffen mit eingebauten, gegenüber Isocyanatgruppen reaktionsfähigen Gruppen gestatten.

Geeignete Verbindungen mit gegenüber Isocyanatgruppen reaktionsfähigen Gruppen sind insbesondere mehrwertige aliphatische Alkohole, die gegebenenfalls Ether- oder Estergruppierungen aufweisen. Auch Hydroxylgruppen aufweisende Polyurethane können bei der Umsetzung als Verbindungen mit gegenüber Isocyanatgruppen reaktionsfähigen Gruppen eingesetzt werden. Die Verbindungen mit gegenüber Isocyanatgruppen reaktionsfähigen Gruppen weisen im allgemeinen ein Molekulargewicht von 62-10 000, vorzugsweise 62-4 000 und insbesondere 62-400 auf.

Setzt man beispielsweise einfache Diole des Molekulargewichtsbereichs 62-400 wie z. B. Ethylenglykol, Propan-1,2-diol, Butan-1,3-diol, Butan-1,4-diol oder Neopentylglykol mit den erfindungsgemäßen Esterisocyanaten unter Einhaltung eines NCO/OH-Äquivalentverhältnisses von 1 : 1 um, so erhält man Urethane, die im Unterschied zu den analog aus 2-Isocyanatoethylmethacrylat hergestellten Verbindungen bei Raumtemperatur flüssig sind und/oder eine verbesserte Verträglichkeit mit anderen olefinisch ungesättigten Monomeren aufweisen. So stellen beispielsweise die Umsetzungsprodukte der oben zuletzt genannten Esterisocyanat-Gemische mit Butan-1,3-diol, Butan-1,4-diol oder Neopentylglykol bei Raumtemperatur Flüssigkeiten dar. Die Umsetzungsprodukte der gleichen Esterisocyanat-Gemische mit mehrwertigen Alkoholen wie z. B. Glycerin oder Trimethylolpropan weisen eine deutlich niedrigere Schmelzviskosität als die analog aus 2-Isocyanatoethylmethacrylat hergestellten Urethane auf.

In den nachfolgenden Beispielen beziehen sich alle Prozentangaben auf Gewichtsprozente.

In den nachfolgenden Beispielen 1-3 wurden folgende Dihydrooxazine bzw. Dihydrooxazin-Gemische eingesetzt :

Dihydrooxazin I

122 g Paraformaldehyd, 1600 ml Essigsäure und 392 g 100 % Schwefelsäure werden 20 Min. auf 60 °C erwärmt. Bei 50 °C gibt man in 10 Min. eine Lösung von 0,5 g Phenothiazin in 212 g Acrylniril hinzu und rührt 30 Min. bei 50 °C nach. Bei 70 °C sättigt man 2 Std. mit einem Gemisch aus 20 % cis-Buten-2, 37 % trans-Buten, 12 % 1-Buten, 7 % iso-Butan, 23 % n-Butan und 1 % nicht identifizierter Kohlenwasserstoffe. Die klare Lösung wird auf Raumtemperatur gekühlt. Das Lösungsmittel wird im Vakuum abdestilliert. Der Rückstand wird unter Kühlung in 6,5 l 15 % Natronlauge und 2 l Methylenchlorid eingerührt, abgesaugt, die organische Phase wird abgetrennt, die wäßrige Phase wird noch zweimal mit Methylenchlorid extrahiert und über Natriumsulfat getrocknet. Die Destillation im Vakuum ergibt 250 g (45 % der Theorie) eines Gemischs aus (i) 90 Gew.-teilen 5,6-Dimethyl-2-vinyl-5,6-dihydrooxazin (cis/trans-Gemisch) und (ii) 10 Gew.-Teilen 6-Ethyl-2-vinyl-5,6-dihydrooxazin. Das Gemisch weist bei 10 mbar einen Siedebereich von 75-78 °C auf.

Dihydrooxazin II

24 g 1,3,5-Trioxan, 320 g Essigsäure und 78 g 100 % Schwefelsäure werden 20 Min. Bei 70 °C gerührt. Bei 50 °C tropft man 53,6 g Methacrylnitril zu, rührt 30 Min. bei 50 °C, sättigt 2 Stunden lang bei

70 °C mit dem oben genannten Olefingemisch und kühlt auf Raumtemperatur, entfernt das Lösungsmittel im Vakuum und rührt den Rückstand unter Kühlung in 2 l 15 % Natronlauge und 500 ml Methylenchlorid ein. Die Phasen werden getrennt, die wäßrige Phase wird zweimal mit Methylenchlorid extrahiert. Man trocknet über Natriumsulfat und destilliert im Vakuum. Man erhält 50 G (41 % der Theorie) einer Mischung aus (i) 90 Gew.-teilen 5,6-Dimethyl-2-isopropenyl-5,6-dihydrooxazin (cis-/trans-Gemisch) und (ii) 10 Gew.-teilen 6-Ethyl-2-isopropenyl-5,6-dihydrooxazin. Das Gemisch weist bei 20 mbar einen Siedebereich von 85-90 °C auf.

Dihydrooxazin III

31 g Paraformaldehyd, 60 g Essigsäure, 98 g 100 % Schwefelsäure und 300 ml Tetrahydrofuran werden 30 Min. am Rückfluß erhitzt. Bei 50 °C gibt man 53 g Acrylnitril zu, rührt 30 Min. bei 50 °C nach und sättigt 2 Stunden unter Rückfluß mit dem oben genannten Olefingemisch. Das Lösungsmittel wird im Vakuum entfernt. Der Rückstand wird unter Kühlung in 1,2 l 15 % Natronlauge und 800 ml Methylenchlorid eingerührt und wie oben beschrieben aufgearbeitet. Die Destillation im Vakuum ergibt 57 g (41 % der Theorie) eines Dihydrooxazin-Gemischs, das bezüglich seiner Zusammensetzung und seinem Siedepunkt dem Dihydrooxazin I entspricht.

Dihydrooxazin IV

31 g Paraformaldehyd, 98 g 100 % Schwefelsäure und 250 ml Essigsäure werden 30 Min. auf 70 °C erwärmt. Bei 50 °C gibt man 53 g Acrylnitril hinzu, rührt 30 Min. bei 50 °C nach. Bei 70 °C tropft man 82 g Cyclohexen zu, rührt 2 Stunden bei 70 °C, rührt unter Kühlung in 4 l 10 % Natronlauge und 1,5 l Methylenchlorid ein und arbeitet wie oben beschrieben weiter auf. Man erhält 59 g (36 % der Theorie) eines Dihydrooxazins der Formel

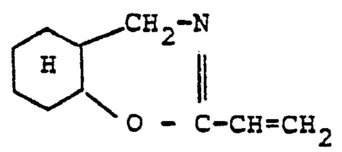

(VII)

mit einem Siedebereich bei 0,1 mbar von 55-57 °C.

Beispiel 1

Pro Stunde werden unter starkem Rühren gleichzeitig Lösungen von 226 g Dihydrooxazin I (bzw. III) in 300 ml Methylenchlorid, von 238 g Phosgen in 800 ml Methylenchlorid und von 217 g Natriumhydroxid in 1 200 ml Wasser in ein Reaktionsgefäß mit Überlauf eingepumpt. Die Temperatur wird bei 0-5 °C gehalten. Die Phasen der überlaufenden Lösung werden getrennt. Man extrahiert die wäßrige Phase noch zweimal mit Methylenchlorid, trocknet die vereinigten organischen Phasen über Natriumsulfat und destilliert im Vakuum. Man erhält pro Stunde 232 g (78 % der Theorie) eines Gemischs aus 1-Isocyanato-2-methyl-but-3-yl-acrylat und 1-Isocyanato-pent-3-yl-acrylat im Gewichtsverhältnis 90 : 10 des Siedebereichs bei 0,1 mbar von 75-77 °C.

Beispiel 2

Innerhalb 10 Min. tropft man gleichzeitig Lösungen von 30 g Phosgen in 80 ml Methylenchlorid, von 29 g eines Dihydrooxazins II in 70 ml Methylenchlorid und von 26 g Natriumhydroxid in 170 ml Wasser unter starkem Rühren in ein Reaktionsgefäß. Die Temperatur wird dabei zwischen 0 und 10 °C gehalten. Die Phasen werden getrennt, die wäßrige Phase wird zweimal mit Methylenchlorid nachextrahiert. Man trocknet die vereinigten organischen Phasen über Natriumsulfat und destilliert im Vakuum. Man erhält 30 g (81 %) eines Gemischs aus 1-Isocyanato-2-methyl-but-3-yl-methacrylat und 1-Isocyanatopent-3-yl-methacrylat im Gewichtsverhältnis 90 : 10 mit einem Siedebereich bei 0,1 mbar von 80-85 °C.

Beispiel 3

Innerhalb von 10 Minuten tropft man gleichzeitig Lösungen von 18 g Phosgen in 50 ml Methylenchlorid, von 20 g des Dihydrooxazins IV in 50 ml Methylenchlorid und von 18 g Natriumhydroxid in 130 ml Wasser unter kräftigem Rühren bei 0 bis 5 °C in ein Reaktionsgefäß ein. Wenn die Zugabe beendet ist, trennt man die Phasen, extrahiert die wäßrige Phase noch zweimal mit Methylenchlorid. Die vereinigten organischen Phasen werden über Natriumsulfat getrocknet und im Vakuum destilliert. Man erhält 17,5 g (70 %) 2-Isocyanatomethylcyclohexylacrylat, $Kp._{0,1}$ 95-97°.

Beispiel 4

122 g Paraformaldehyd, 212 g Acrylnitril, 392 g 100 % Schwefelsäure werden wie bei der Herstellung von Dihydrooxazin I beschrieben mit dem dort genannten Olefingemisch zur Reaktion gebracht. Nach dem Abdestillieren des Lösungsmittels wird der Rückstand in 800 ml Methylenchlorid gelöst. Diese Lösung wird innerhalb 3 Stunden bei 0-5 °C unter guter Durchmischung gleichzeitig mit Lösungen von 594 g Phosgen in 2 000 ml Methylenchlorid und von 1 000 g Natriumhydroxid in 6 000 ml Wasser in ein Reaktionsgefäß (600 ml) mit Überlauf eingepumpt. Die Phasen der überlaufenden Lösung werden getrennt. Man extrahiert die wäßrige Phase noch zweimal mit Methylenchlorid und trocknet die vereinigten organischen Phasen über Natriumsulfat. Die Destillation im Vakuum ergibt 329 g (45 %, bezogen auf Acrylnitril) des in Beispiel 1 beschriebenen Isocyanatoacrylat-Gemischs.

Beispiel 5

Zu 27 g Butan-1,4-diol, 25 mg Ionol und 50 mg Dibutylzinndilaurat werden bei 70 °C 109,8 g des Esterisocyanatgemischs aus Beispiel 1 zugetropft. Man rührt 2 Stunden bei 70 °C nach. Man erhält eine quantitative Ausbeute des entsprechenden Diurethans mit einer Viskosität von 11 100 mPas bei 23 °C.

Beispiel 6

Aus 27 g Butan-1,4-diol, 90 g 2-Isocyanatoethylmethacrylat werden in Gegenwart von 25 mg Ionol und 50 mg Dibutylzinndilaurat wie in Beispiel 5 beschrieben in quantitativer Ausbeute das entsprechende Diurethan hergestellt. Das Umsetzungsprodukt weist einen Schmelzpunkt von 50 °C auf.

**Patentansprüche**

1. Gegebenenfalls als Isomerengemische vorliegende, ungesättigte Esterisocyanate, dadurch gekennzeichnet, daß sie im wesentlichen
zu 0-95 Gew.-%, bezogen auf Gesamtgemisch, aus Verbindungen der Formel

$$OCN-CH_2-R_1-O-CO-\overset{R_2}{C}=CH-R_3 \qquad (I)$$

zu 0 bis 70 Gew.-%, bezogen auf Gesamtgemisch, aus Verbindungen der Formel

$$OCN-CH_2-R_1'-O-CO-\overset{R_2}{C}=CH-R_3 \qquad (II)$$

und zu 0-70 Gew.-%, bezogen auf Gesamtgemisch, aus Verbindungen der Formel

$$OCN-CH_2-R_1''-O-CO-\overset{R_2}{C}=CH-R_3 \qquad (III)$$

bestehen, wobei sich die genannten Prozentsätze jeweils zu 100 ergänzen und wobei

$R_1$, $R'_1$ und $R''_1$ entweder für den gleichen Rest stehen und jeweils einen gegebenenfalls Alkylsubstituierten 1,2-Cycloalkylen-Rest mit insgesamt 5 bis 10 Kohlenstoftatomen bedeuten, oder wobei $R_1$ für einen 2,3-Butylen-Rest, $R'_1$ für einen geradkettigen 1,2-Butylen-Rest und $R''_1$ für einen 1,2-Isobutylen-Rest stehen,

$R_2$ und $R_3$ für gleiche oder verschiedene Reste stehen und jeweils Wasserstoff oder eine Methylgruppe bedeuten.

2. Ungesättigte Esterisocyanate gemäß Anspruch 1 der Formel

$$OCN-CH_2-R_1-O-CO-\overset{R_2}{C}=CH-R_3 \qquad (I)$$

wobei

$R_1$ für einen gegebenenfalls Alkyl-substituierten 1,2-Cycloalkylen-Rest mit insgesamt 5 bis 10 Kohlenstoffatomen steht,

$R_2$ für Wasserstoff oder eine Methylgruppe steht und

$R_3$ für Wasserstoff steht.

3. Isomerengemische darstellende, ungesättigte Esterisocyanate gemäß Anspruch 1, dadurch

gekennzeichnet, daß sie zu

60 ± 10 Gew.-% aus threo-1-Isocyanato-2-methyl-but-3-yl-(meth)acrylat
30 ± 10 Gew.-% erythro-1-Isocyanato-2-methyl-but-3-yl-(meth)acrylat und zu
10 ± 5 Gew.-% 1-Isocyanatopent-3-yl-(meth)-acrylat

bestehen.

4. Verfahren zur Herstellung von, gegebenenfalls als Isomerengemische vorliegenden, ungesättigten Esterisocyanate gemäß Anspruch 1 durch Umsetzung von gegebenenfalls als Säureaddukte vorliegenden Dihydrooxazinen mit Phosgen bei — 20 bis + 20 °C in einem mit Wasser nicht mischbaren Lösungsmittel in Gegenwart einer wäßrigen Lösung einer Base, dadurch gekennzeichnet, daß man als Dihydrooxazine solche verwendet, die sich im wesentlichen
zu 0-95 Gew.-% aus Verbindungen der Formel

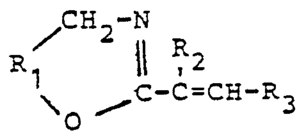

(IV)

zu 0-70 Gew.-% aus Verbindungen der Formel

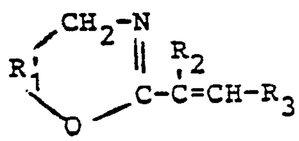

(V)

und zu 0-70 Gew.-% aus Verbindungen der Formel

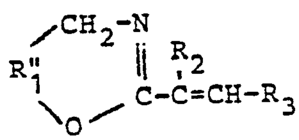

(VI)

zusammensetzen, wobei sich die Prozentsätze jeweils zu 100 ergänzen, und wobei $R_1$, $R'_1$, $R''_1$, $R_2$ und $R_3$ die in Anspruch 1 genannte Bedeutung haben.

5. Verwendung der ungesättigten Esterisocyanate gemäß den Ansprüchen 1 bis 3 als Ausgangsmaterialien bei der Herstellung von olefinisch ungesättigten Oligourethanen.

## Claims

1. Unsaturated ester isocyanates which are optionally present in the form of isomer mixtures, characterised in that they substantially consist of
0-95 % by weight, based on the total mixture, of compounds of the formula

$$OCN-CH_2-R_1-O-CO-\overset{R_2}{\overset{|}{C}}=CH-R_3 \quad (I)$$

0 to 70 % by weight, based on the total mixture, of compounds of the formula

$$OCN-CH_2-R'_1-O-CO-\overset{R_2}{\overset{|}{C}}=CH-R_3 \quad (II)$$

and 0-70 % by weight, based on the total mixture, of compounds of the formula

$$OCN-CH_2-R''_1-O-CO-\overset{R_2}{\overset{|}{C}}=CH-R_3 \quad (III)$$

the abovementioned percentages in each case totalling 100 and wherein

$R_1$, $R'_1$, and $R''_1$ either represent the same radical and each denotes optionally alkyl-substituted 1,2-cycloalkylene radical with a total of 5 to 10 carbon atoms, or wherein $R_1$ represents a 2,3-butylene radical, $R'_1$ represents a straight-chain 1,2-butylene radical and $R_1''$ represents a 1,2-isobutylene radical,

$R_2$ and $R_3$ represent identical or different radicals and each denotes hydrogen or a methyl group.

2. Unsaturated ester isocyanates according to Claim 1 of the formula

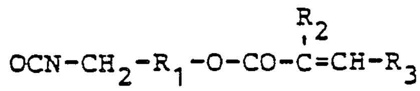

(I)

wherein

$R_1$ represents an optionally alkyl-substituted 1,2-cycloalkylene radical with a total of 5 to 10 carbon atoms,

$R_2$ represents hydrogen or a methyl group and

$R_3$ represents hydrogen.

3. Unsaturated ester isocyanates, which are in the form of isomer mixtures, according to Claim 1, characterised in that they consist of

60 ± 10 % by weight of threo-1-isocyanato-2-methyl-but-3-yl-(meth)acrylate,
30 ± 10 % by weight of erythro-1-isocyanato-2-methyl-but-3-yl-(meth)acrylate and
10 ± 5 % by weight of 1-isocyanatopent-3-yl-(meth)acrylate.

4. Process for the production of unsaturated ester isocyanates according to Claim 1, which are optionally present in the form of isomer mixtures, by reacting dihydroxazines, which are optionally present in the form of acid adducts, with phosgene at — 20 to + 20 °C in a water-immiscible solvent in the presence of an aqueous solution of a base, characterised in that the dihydrooxazines used are those which are substantially composed of

0-95 % by weight of compounds of the formula

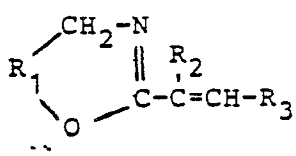

(IV)

0-70 % by weight of compounds of the formula

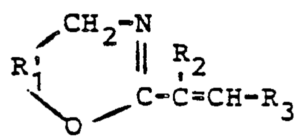

(V)

and 0-70 % by weight of compounds of the formula

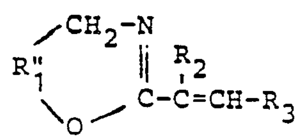

(VI)

the percentages in each case totalling 100, and wherein $R_1$, $R'_1$, $R''_1$, $R_2$ and $R_3$ have the meaning mentioned in Claim 1.

5. Use of the unsaturated ester isocyanates according to Claims 1 to 3 as starting materials in the production of olefinically unsaturated oligourethanes.

**Revendications**

1. Isocyanato-esters insaturés, existant éventuellement sous forme de mélanges d'isomères, caractérisés en ce qu'ils sont principalement formés en proportion de 0 à 95 % en poids, par rapport au mélange total, de composés de formule

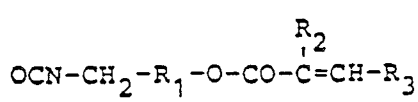

(I)

en proportion de 0 à 70 % en poids, par rapport au mélange total, de composés de formule

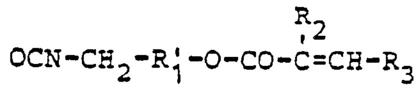

(II)

et en proportion de 0 à 70 % en poids, par rapport au mélange total, de composés de formule

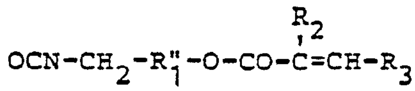

(III)

les pourcentages mentionnés formant un total de 100 dans chaque cas et

$R_1$, $R'_1$ et $R''_1$ représentent le même reste et désignent chacun un reste 1,2-cycloalkylène éventuellement substitué par un groupe alkyle, avec au total 5 à 10 atomes de carbone, ou bien $R_1$ est un reste 2,3-butylène, $R'_1$ est un reste 1,2-butylène à chaîne droite et $R''_1$ est un reste 1,2-isobutylène,

$R_2$ et $R_3$ sont des restes identiques ou différents et représentent chacun l'hydrogène ou un groupe méthyle.

2. Isocyanato-esters insaturés suivant la revendication 1, de formule

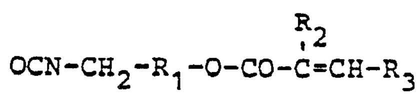

(I)

dans laquelle

$R_1$ est un reste 1,2-cycloalkylène éventuellement substitué par un groupe alkyle, totalisant 5 à 10 atomes de carbone,

$R_2$ est l'hydrogène ou un groupe méthyle, et

$R_3$ est l'hydrogène.

3. Isocyanato-esters insaturés formant des mélanges d'isomères suivant la revendication 1, caractérisés en ce qu'ils sont formés, en proportion de

60 ± 10 % en poids, de thréo-1-isocyanato-2-méthyl-but-3-yl-(méth)acrylate

en proportion de

30 ± 10 % en poids, d'érythro-1-isocyanato-2-méthyl-but-3-yl-(méth)-acrylate

et en proportion de

10 ± 5 % en poids, de 1-isocyanatopent-3-yl-(méth)acrylate.

4. Procédé de production d'isocyanato-esters insaturés existant éventuellement sous forme de mélanges d'isomères suivant la revendication 1 par réaction de dihydro-oxazines se présentant éventuellement sous la forme de produits d'addition d'acides, avec le phosgène à une température de — 20 à + 20 °C dans un solvant non miscible à l'eau en présence d'une solution aqueuse d'une base, caractérisé en ce qu'on utilise des dihydro-oxazines qui sont formées principalement en proportion de 0 à 95 % en poids, de composés de formule

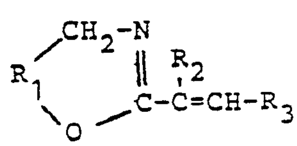

(IV)

en proportion de 0 à 70 % en poids, de composés de formule

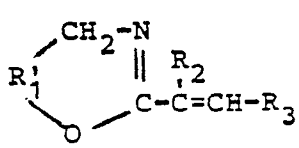

(V)

et en proportion de 0 à 70 % en poids, de composés de formule

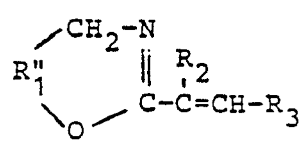

(VI)

les pourcentages représentant dans chaque cas un total de 100, et $R_1$, $R'_1$, $R''_1$, $R_2$ et $R_3$ ont les définitions indiquées dans la revendication 1.

5. Utilisation des isocyanato-esters insaturés suivant les revendications 1 à 3 comme matières de départ dans la production d'oligo-uréthannes à insaturation oléfinique.